# EUROPEAN PATENT APPLICATION

(11) **EP 1 972 339 A1**
(43) Date of publication of application: **24.09.2008**
(21) Application number: 08250945.6
(22) Date of filing: 18.03.2008
(51) Int. Cl.: A61K 31/401, A61P 7/04, A61K 33/14

(54) **Haemostatic Compositions**

(30) Priority: 21.03.2007 GB 0705437
(71) Applicant: Nozotec AB, 412 66 Göteborg (SE)
(72) Inventor: Petruson, Björn, 412 66 Göteborg (SE)
(74) Representative: Towler, Philip Dean

(57) **Abstract**

The invention relates to a composition comprising: (a) one or more aminoacids selected from glycine, proline and hydroxyproline; (b) a source of calcium ions; and (c) water. The invention also extends to a dry composition comprising the aforementioned components (a) and (b). The compositions of the invention are useful for arresting bleeding of humans and other animals, in particular for accelerating haemostasis at the site of incisions made during surgical procedures.

## Description

The present invention relates to a composition that is useful for arresting bleeding of humans and other animals. The invention has general utility but is particularly useful for accelerating haemostasis at the site of incisions made during surgical procedures.

### Background of the Invention

Whilst bleeding is a generally undesirable consequence of physical trauma, it is usually an inevitable part of surgery, when it may arise as a result of incisions made either at the ultimate site of the surgical procedure or in order to gain physical access to that site. Bleeding may be arterial, venous or capillary, and these are conventionally treated in different ways.

Arterial bleeding can be treated using electric coagulation (cauterization), or making sutures in the surrounding tissue, around the vessel, or in the vessel wall. Bleeding from large veins can be similarly treated; it is also possible to exert physical pressure at the site of the bleeding in order to staunch the blood flow.

Bleeding from small venous vessels and capillaries is most commonly treated during surgery by physically pressing against the bleeding area a compress soaked in saline solution.

Bleeding from small venous vessels and capillaries can also be treated using a variety of different agents that facilitate coagulation. For example, Spongostan^{®} is a prefabricated absorbable sponge product comprising gelatine powder (commercially available from Ferrosan A/S, Denmark). Surgicel^{®} is an absorbable haemostatic gauze consisting of a matrix of oxidised regenerated cellulose (commercially available from Johnson & Johnson, New Jersey, USA). Haemostatis can also be promoted biochemically, for example covering the bleeding area with a mixture of the coagulation factors thrombin and fibrinogen, leading to the formation of an artificial clot. According to all these methods the haemostatic agents are left *in situ* to be absorbed by the body over a period of one or several weeks.

Various haemostatic compositions have been described in the art. For example, JP-A-2002060341 discloses a haemostatic agent comprising a calcium salt of a nucleic acid such as DNA. This is said to exhibit a rapid coagulation capable of stopping arterial bleeding, as well as adhering to tissues and being biodegradable.

### Summary of the invention

The inventor has surprisingly discovered a haemostatic composition which is of particular use in arresting arterial, venous and capillary bleeding during surgery.

According to the invention there is provided a composition comprising:
(a) one or more aminoacids selected from glycine, proline and hydroxyproline;
(b) a source of calcium ions; and
(c) water.

Additional materials may also be used that are conventional in the art of formulating such compositions, as discussed in more detail below.

The invention also provides a process for making the novel composition, comprising mixing water with the components (a) and (b) above, in any desired order.

In a yet further aspect, the invention provides a composition of the invention for use in therapy or surgery, especially for use in arresting arterial, venous or capillary bleeding.

Although the compositions are used for therapy in aqueous form, it is also possible to prepare compositions of the dry ingredients (a) and (b) above, and then prepare aqueous compositions at a subsequent time, prior to use. Accordingly, in a still further aspect, the present invention provides a dry composition comprising:
(a) one or more aminoacids selected from glycine, proline and hydroxyproline;
   and
(b) a source of calcium ions.

### Detailed description of the invention

The nature and role of the essential components of the compositions of the invention are now described in more detail.

### a) Aminoacids

The first component in the composition comprises one or more of the aminoacids glycine, proline and hydroxyproline. All three of these amino acids can be produced by the human body itself. Glycine is the simplest of all the 20 amino acids and is involved in a great variety of metabolic reactions, including the formation of proteins in the human body. Glycine, proline and hydroxyproline are all important building blocks of the protein collagen found in many human and animal cells and may play a role in the restoration of blood vessel walls.

Glycine, proline and hydroxyproline are widely commercially available materials. Proline and hydroxyproline have more than one stereoisomerism form. The present invention relates to all isomeric forms of these compounds, including mixtures thereof.

Glycine, proline and hydroxyproline may comprise up to 99.995 weight % of the total dry ingredients in the compositions of the inventions. Preferably, glycine constitutes the largest part of the aminoacid component (a), preferably 40-80 wt.%, more preferably 50-70 wt.%, most preferably around 60 wt.%. It is also preferred that proline and hydroxyproline each constitutes no more than 30 wt.% of aminoacid component (a). In a preferred embodiment, all three of glycine, proline and hydroxyproline are present in the composition. In one especially preferred embodiment, the weight ratio of glycine:proline:hydroxyproline in component (a) is about 3:1:1.

In the aqueous compositions of the invention, the concentration of glycine may vary from 1 to 200 g dry weight per litre of solution. For use in a surgical context, the concentration of glycine is typically towards the upper end of this range, i.e. 50 to 200 g/l, preferably from 55 to 100 g/l, more preferably about 60 g/l. For use in a domestic setting, the concentration of glycine is typically at the lower end of this range, i.e. 5 to 20 g/l, preferably from 5.5 to 10 g/l, more preferably about 6 g/l.

For each of proline and hydroxyproline, the concentration is typically from 0.5 to 50 g/l. For use in a surgical context, the concentration is preferably from 10 to 40 g/l, more preferably about 20 g/l. For use in a domestic setting, the concentration is preferably from 1 to 4 g/l, more preferably about 2 g/l.

### b) Source of calcium ions

The other essential component is a source of calcium ions (Ca²⁺). Calcium ions are implicated in biological coagulation processes. When a blood vessel wall is damaged, haemostasis occurs in order to arrest the blood loss. In the first step, there is constriction of the surrounding vessel. Primary haemostasis then occurs, in which denuded collagen affects the platelets (thrombocytes) and causes them to aggregate. In the third step a fibrin clot forms which occludes the damaged vessel wall, stopping the bleeding. Soluble fibrin is converted to an insoluble fibrin clot, which can withstand mechanical forces more effectively, thereby reducing the risk of rebleeding. In the final step, the clot dissolves over a period of around 10 days, during which time the vessel wall heals so that the vessel can function normally again. Without wishing to be bound by any theory, it is believed that the second and third steps of haemostasis proceed more rapidly in the presence of calcium ions.

The calcium ions may be present in any convenient form, but are preferably in the form of a soluble salt, for example the chloride, or another salt, e.g. acetate, phosphate, carbonate or gluconate. Calcium chloride is most preferred.

The total amount of calcium ions is typically from 0.005 to 0.1 % by weight of the total dry ingredients, preferably from 0.01 to 0.05 wt.% , more preferably from 0.025 to 0.040 wt.%, expressed as the weight of Ca²⁺ ions only, based on the total weight of dry ingredients.

In the aqueous compositions of the invention, the concentration of calcium ions is typically from 10 to 200 mg per litre, preferably from 20 to 100 mg/l, more preferably from 40 to 70 mg/l, most preferably about 58 mg/l.

### Other components

In addition to the essential components discussed above, other additional materials may also be used that are conventional in the art of formulating such compositions. By way of example only, these may include pH adjusting agents (acids, alkalis, buffers), preservatives (e.g. methargen, propagin), antioxidants, excipients, carriers and the like.

### Process conditions

In order to formulate the compositions of the invention for clinical use, the following steps are carried out.

A source of calcium ions, e.g. calcium chloride, is dissolved in (sterilised) water. Before or after mixing with the source of calcium ions, a suitable amount of acid, alkali or buffer material may be added to the water in order to ensure that the final solution has a suitable pH, e.g. in the range 6.5-7.5. One or more of the aminoacids glycine, proline and hydroxyproline are then added with stirring until dissolution is complete. Any other components may be added at a suitable time. The pH of the solution may be checked to verify that it is within the preferred range of 6.5-7.5.

The order in which the components are combined may be varied from that described in the foregoing paragraph. For example, the dry ingredients may firstly be mixed together, and then water added in a suitable quantity.

If desired, the solutions obtained by the above process may be converted to a gel, cream or similar for topical use, in a manner known to the person skilled in the art.

The preparation of the compositions may be carried out at any suitable temperature. However, preparation at room temperature is most convenient. In order to avoid undesirable contamination of the compositions, the preparation should be effected in a sterile environment.

The conditions of the foregoing process can be varied and optimised in a manner familiar to a person skilled in the art.

### Manner of administration

In use, the compositions are applied to the area of bleeding in any convenient manner. For surgical use, this may be by sprinkling, spraying or pouring over the bleeding area a composition in the form of an aqueous solution. Alternatively, a compress may be soaked with the aqueous solution and then pressed against the site of bleeding. Prefabricated compresses may be made by soaking compresses with the aqueous solution in advance, under sterile conditions. Exerting pressure at the site of the bleeding constricts the bleeding vessel, and thereby improves the conditions for the first step in haemostasis, as described above. Compresses intended for use in a surgical context are preferably soaked with an aqueous composition of the invention having concentrations of the aminoacid components at the higher end of the typical ranges, as discussed above.

Alternatively, a composition of the invention may be applied in the form of a gel or cream. Such formulations are particularly suitable for topical use in a domestic setting, for example by application to small wounds resulting from scratching the skin or from minor cuts caused by sharp items. In these formulations, the aminoacid ingredients may desirably be present in amounts at the lower end of the ranges as discussed above.

Plasters (of construction types known in the art) for domestic or clinical use may be impregnated with, or include a (e.g. woundside) layer of an aqueous composition of the invention (including a cream or gel).

Compresses and plasters comprising (e.g. impregnated with) an aqueous composition of the invention constitute another aspect of the present invention.

Compositions of the invention may also be incorporated into a dentifrice formulated in a manner conventional in the art. Such dentifrices constitute a further aspect of the present invention. Bleeding from the gums during toothbrushing is a relatively common problem. Dentifrices incorporating compositions of the invention are of particular use in preventing or arresting bleeding from the gums.

If desired, the end user may add a suitable quantity of water to a dry powder composition of the invention to form an aqueous solution shortly prior to the use thereof.

An advantage of the compositions of the invention is that they are well tolerated clinically because the essential components (a) and (b) are naturally present in the human or animal body and are readily absorbed.

### EXAMPLE

The following Example of the manufacture of a composition of the invention is provided in order to illustrate the invention only, and is not to be taken as limiting its scope.

520 g pure and sterile water and 380 g of 1 M sodium hydroxide solution were placed together in a glass vessel. 185 mg of calcium chloride (CaCl₂.2H₂O) was added, and the mixture was stirred until the calcium chloride had dissolved. 60 g Glycine, 20 g proline and 20 g hydroxyproline were then added with stirring until dissolution was complete. All the foregoing steps were carried out at room temperature under sterile conditions. The pH of the solution was checked to ensure that it was within the desired range of 6.5-7.5.

## Claims

1. A composition comprising:
(a) one or more aminoacids selected from glycine, proline and hydroxyproline;
(b) a source of calcium ions; and
(c) water.

2. A composition of claim 1 wherein component (a) comprises 40-80 wt.% glycine.

3. A composition of claim 1 or claim 2 wherein component (a) comprises no more than 30 wt.% proline and no more than 30 wt.% hydroxyproline.

4. A composition of any preceding claim wherein component (a) comprises a mixture of all three of glycine, proline and hydroxyproline.

5. A composition of claim 4 wherein component (a) comprises about 60 wt.% glycine, about 20 wt.% proline and about 20 wt.% hydroxyproline.

6. A composition of any preceding claim wherein the source of calcium ions is calcium chloride.

7. A composition of claim 16 wherein the concentration of calcium ions is from 20 to 100 mg/l.

8. A composition of any preceding claim in the form of a topical gel or cream.

9. A process for making a composition as claimed in any of claims 1 to 8, comprising mixing water with (a) one or more aminoacids selected from glycine, proline and hydroxyproline, and (b) a source of calcium ions, in any desired order.

10. A composition as claimed in any of claims 1 to 8 for use in therapy or surgery.

11. A composition as claimed in any of claims 1 to 8 for,use in arresting arterial, venous or capillary bleeding.

12. A compress or plaster impregnated with a composition as claimed in any of claims 1 to 8.

13. A dentifrice comprising a composition as claimed in any of claims 1 to 8.

14. A dry composition comprising:
(a) one or more aminoacids selected from glycine, proline and hydroxyproline;
and
(b) a source of calcium ions.
